Europäisches Patentamt

European Patent Office

Office européen des brevets

(19)

(11) Publication number: **0 397 459**
**A1**

# EUROPEAN PATENT APPLICATION

(21) Application number: **90304959.1**

(51) Int. Cl.⁵: **A61M 25/01**

(22) Date of filing: **08.05.90**

(30) Priority: **08.05.89 US 350678**

(43) Date of publication of application:
**14.11.90 Bulletin 90/46**

(84) Designated Contracting States:
**DE FR GB NL**

(71) Applicant: **MEDTRONIC, INC.**
**7000 Central Avenue N.E.**
**Minneapolis Minnesota 55432-3576(US)**

(72) Inventor: **Wolff, Rodney G.**
**468 West Eagle Lake Drive**
**Maple Grove, MN 55369(US)**
Inventor: **Hull, Vincent W.**
**1250 72nd Avenue N.E.**
**Fridley, MN 55432(US)**

(74) Representative: **Piesold, Alexander J. et al**
**Frank B. Dehn & Co. Imperial House 15-19**
**Kingsway**
**London WC2B 6UZ(GB)**

(54) Catheter.

(57) A catheter (10) has a torque cable (12) having a first coil (14) wound at a first angle and a second coil (16) concentric to the first coil and wound at a second, opposite angle. Rotation applied at a proximal end (20) of the cable (12) results in generally equivalent rotation at its distal end (22). A sheath (50) is mounted on the cable (12) and is connected to a balloon (52) adjacent the distal end (22).

FIG.1.

# CATHETER

This invention relates to catheters (e.g. PTCA catheters) for insertion in body vessels, such as for clearing arterial stenosis.

The opening of a coronary vessel partly occluded by stenosis 15 now an accepted alternative to open heart bypass surgery.

Early balloon angioplasty is typified by the work of Dr. Gruntzig as illustrated in United States Patent No. 4,195,637. Dr. Gruntzig used both dual lumen side-by-side catheters and coaxial catheters which were inserted over a guide wire into an artery.

Later, it became common to use a single lumen device in which a balloon was mounted directly on a guide wire, such as United States Patent No. 4,307,722 to Evans. This basic design was also followed by Samson in United States Patent No. 4,582,181.

To provide therapy, the catheter must be fed through the artery to the point of occlusion. This can be a tortuous path. In the usual technique, the catheter is inserted in femoral artery and threaded up to the coronary arteries on the heart. Many tight turns must be traversed in order to accomplish this, especially in the destination area in the coronary arteries.

Many approaches have been tried to obtain steerability in guide wires or in catheters in order to move about these various curves. In the catheters which slide over a guide wire, it is well known to use guide wires with flexible tips to help accomplish such turns.

In the case of balloons attached to guide wires like Evans, it is difficult to apply enough torque to turn a catheter to help guide it around these curves. For example, the Samson approach used a wire to transmit the torque. This results in a relatively stiff device. More importantly, this guide wire turns the catheter tip to guide it, but does not rotate the entire catheter. Since the catheter remains radially stationary within the artery while the internal guide wire twists, the balloon tends to twist as the guide wire within it turns. There are practical limits, called rotation limiters, on how much torque can be applied before the balloon is twisted to the point where it binds and the wire cannot be turned any further. Of course, it is difficult to inflate such a twisted balloon. Commonly this prior art device allows two rotations in a clockwise direction from a neutral position before such binding.

Various techniques have been tried for strengthening catheter walls to provide torque. These include helically wound wires, which did not provide sufficient torque. Additionally, they include molding of mesh or coils into catheter walls, such as in United States Patent 3,416,531 to Edwards, issued December 17, 1968. These techniques stiffened plastic catheters somewhat, but, of necessity, reduced flexibility.

What is needed is a catheter that is flexible enough to negotiate extremely tortuous vessels and reduce the vessel trauma associated with stiff catheters, and that can efficiently transmit torque from the proximal end outside of the human body through the catheter to the distal tip without twisting the balloon and rendering it uninflatable for therapy.

In one broad aspect the present invention is a balloon angioplasty catheter having torque transmitting means for providing substantially 1:1 correspondence between torque applied to the proximal end of the catheter and resulting torque at the distal tip.

Viewed from another aspect the invention provides a medical catheter for insertion in a bodily organ comprising:
a torque-transmitting cable having a proximal end and a distal end for insertion and having a longitudinal axis;
the cable comprising at least a first wire wound in a first coil at a first angle in a first direction, and a second wire wound in a second coil, generally concentric to the first coil in a second opposite direction at a second generally opposite angle;
the second coil being wound against the first coil, forming the torque-transmitting cable whereby rotational force applied at the proximal end results in generally equivalent rotational movement of the distal end; and
whereby the cable is flexible in all directions about the axis except for rotational torque means mounted generally adjacent the distal end for interacting with the bodily organ.

Viewed from another aspect the invention provides an angioplasty device comprising:
a torque-transmitting cable having a longitudinal axis with a proximal end and a distal end, a first wire coil wound at a first angle to the axis forming a central lumen, a second coil wound around the first coil at a second generally opposite angle to the axis, and a third coil wound about the second coil at a third angle generally opoposite the second angle, the three coils forming the cable so that rotational force applied to the proximal end is generally completely transmitted to the distal end, while the cable is flexible in all other directions;
a generally fluid-tight flexible,
non-torque-transmitting sheath mounted over the cable; a tip wire mounted on the distal end of the cable and extending distally from the cable;

a balloon mounted, at its proximal end, on the sheath having an interior open to the central lumen, the balloon having a distal end extending over the tip wire;

means for attaching the distal end of the balloon to the tip wire.

A preferred embodiment comprises a hollow torque cable wound of first and second wire coils with a central lumen. The coils are tightly wound. The first and second coils are wound at opposite angles to the longitudinal axis of the catheter. The coils form a lumen through the center of the cable. catheter. The coils form a lumen through the center of the cable.

At a distal end of the cable, a segment of wire is preferably bonded. At the distal end of the wire a spring coil guide wire tip may be attached.

The torque cable may be encased in a plastic sheath which preferably has a loose fit about the torque cable. Attached to the sheath there may be a balloon mounted to the sheath generally adjacent to the distal end of the torque cable. The balloon is preferably made of inelastic material so that it has a predetermined shape. The preferable shape is one that has a gradual taper on the distal end and a sharper taper on the proximal end. The balloon then has three general segments: a proximal portion tapering from the sheath outward in a generally frustroconical shape at a first angle to the longitudinal axis of the catheter, a second generally cylindrical middle portion, and a third frustroconical distal portion tapering at a smaller angle to the longitudinal axis. The gradual, narrow taper of the distal portion of the balloon allows easier entry into openings in an occlusion. The balloon may act as a wedge for opening a lesion.

A handle is preferably attached to the proximal end of the torque cable for applying torque to rotate the cable, and thus the entire catheter.

Certain embodiments of the invention will now be described by way of example and with reference to the accompanying drawings, in which:-

Fig. 1 is a cross-sectional view of the distal portion of a catheter constructed according to the present invention.

Fig. 2 is a reduced side plan view of a rotator handle of the proximal end of the catheter of Fig. 1.

Fig. 3 is an end view taken on arrow 3 of Fig. 2.

Fig. 4 is an enlarged cross-sectional view taken on line 4-4 of Fig. 2.

Fig. 5 is an illustration of an alternative embodiment of the catheter according to the present invention.

Fig. 6 is an illustration of yet another alternative embodiment of the catheter according to the present invention.

Fig. 7 is an illustration of an alternative embodiment of the catheter according to the present invention.

Fig. 8 is an illustration of an alternative embodiment of the catheter according to the present invention.

A catheter 10 constructed according to the present invention is based upon torque cable 12. Torque cable 12 is constructed of at least two layers of wound wire. The illustrated embodiment of torque cable 12 includes three layers.

First coil or layer 14 of cable 12 is coiled, preferably tightly, and wound at a first angle to a longitudinal axis through catheter 10. Second coil or layer 16 is wound, preferably tightly, around first coil 14 at a second opposite angle to the longitudinal axis. In this embodiment, a third coil or layer 18 is wound around second coil 16 at a third angle opposite to the second angle.

Layers 14, 16 and 18 may be composed of single-filar coils or ribbons, or multifilar coils or ribbons, in order to obtain the desired degree of stiffness and flexibility to overall cable 12.

In the illustrated embodiment, layer 18 is a flat ribbon coil made of wire with a rectangular cross section. First and second coils 14 and 16 are made of wire with a circular cross section. In the illustrated embodiment, the wire of first and second coils 14 and 16 is made of stainless steel, and has a diameter of 0.004 inches (0.10 mm). The ribbon coil is made of stainless steel and has a rectangular cross section of 0.004 by 0.010 inches (0.10 by 0.25 mm).

Torque cable 12 is very flexible due to its wound-wire construction. Cable 12 may freely bend in all directions. However, torque applied to the proximal end of torque cable 12 is efficiently, and virtually totally, transferred to its distal end.

The preferred torque cable 12 is formed by a process such as winding first coil 14 around a mandrel and then winding successive second and third coils 16 and 18 around first coil 14. The mandrel is then removed to form a lumen 19. A suitable cable for use in practicing the present invention is available from Lake Region Manufacturing Company, Inc.

The torque cable 12 is preferably stronger and more rigid near its proximal end 20 than near its distal end 22. In the illustrated embodiment, the torque cable is approximately 135 cm long. The flat ribbon coil 18 extends for approximately 120 cm from proximal end 20, so that it ends approximately 15 cm before the distal end 22 of torque cable 12. This reduced diameter provides more flexibility in the distal area 22 and allows a lower profile for working the smaller arteries.

A wire 24 is attached, preferably by brazing, to distal end 22 of torque cable 12. In the illustrated embodiment, the wire is approximately 50 mm long. In this embodiment, the wire is constructed of stainless steel and is .006 inches (0.15 mm) in diameter. Wire 24 has a distal tapered portion 26 of smaller diameter. At the distal end of tapered portion 26 of wire 24, the wire is flattened into shaping ribbon 28. A spring guide 30 made of platinum coiled wire is mounted to wire 24 over tapered portion 26 and ribbon 28. A rounded tip 32 is attached, preferably by brazing, to a distal end of spring guide 30. Also, the distal tip of ribbon 28 is attached to the center of tip 32.

At the proximal end 20 of torque cable 12, there is attached a control means 38 for rotating the torque cable 12. Control means 38 includes a handle 39 which, at its proximal end, has a luer fitting 40 for attachment of other medical equipment such as inflation/deflation devices. A lumen 41 extends through handle 39. Attached to the distal end of handle 39 is rotator 42 which includes body 43 and knob 44. Various commercial parts are available to perform the functions of rotator 42, such as products available from Medex Inc. which are used in medical valves and stopcocks. Knob 44 is attached to body 43. As shown in Fig. 4, the proximal end 20 of torque cable 12 is fixedly mounted in knob 44. Therefore, when rotator 42 is turned, torque cable 12 turns with it. Lumen 41 extends through body 43 and knob 44 into the lumen in torque cable 12.

In using the catheter, a stylet 48 may be employed to stiffen and add pushability to torque cable 12 during various parts of the medical procedure. Stylet 48 may be advanced through the lumen 41 of handle 39 and lumen 19 of torque cable 12 to whatever position the physician wishes to make more rigid and create in effect a variable stiffness aspect to catheter 10. Stylet 48 may be advanced all the way to proximal end of wire 24 and used to help push the balloon across a tight stenosis.

Torque cable is encased in a plastic sheath 50, which is preferably formed of irradiated LDPE. In the preferred embodiment, sheath 50 has generally a rather loose fit on torque cable 12. Although a sheath tightly fit to cable 12 will still allow it to operate, better torque transmission is achieved if there is some relative movement allowed of the components of torque cable 12 within sheath 50.

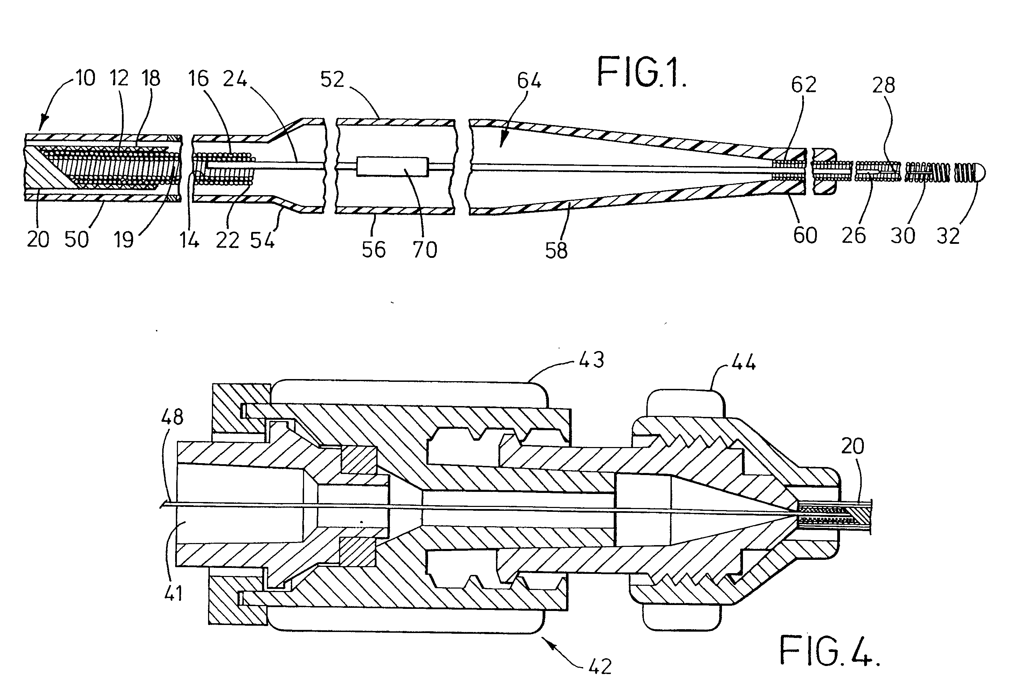

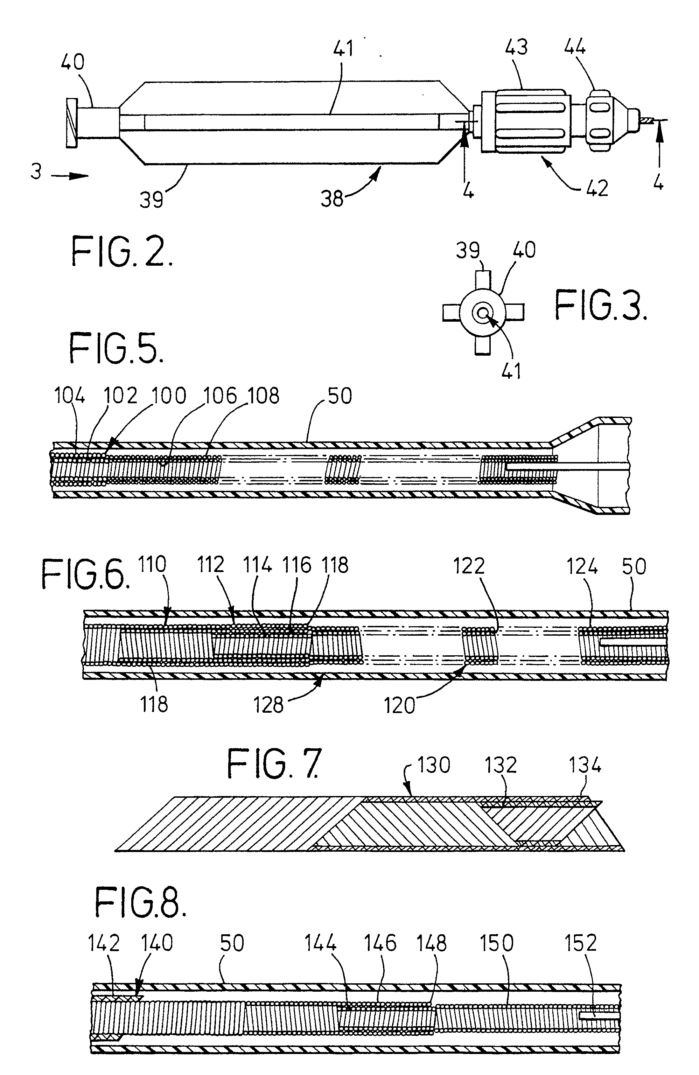

Near distal end 22 of torque cable 12 a balloon 52 is mounted on sheath 50. Balloon 52 may be formed integral with sheath 50, as is known in the prior art, or may be attached by adhesive or other means. Balloon 52 is preferably made of inelastic material so that it inflates to the shape illustrated in Fig. 1. The preferred embodiment of balloon 52 includes a proximal segment 54, a generally cylindrical center segment 56 and a distal segment 58.

Proximal segment 54 is a generally frustroconical shape expanding outwards from the diameter of sheath 50. Walls of segment 54 are at a first angle to the longitudinal axis of catheter 10. Middle segment 56 is generally cylindrical.

Distal segment 58 of balloon 52 is also frustroconical in shape and tapers from the diameter of middle segment 56 down to the tip segment 60. Distal segment 58 tapers at a much smaller angle relative to the longitudinal axis of balloon catheter 10 than does proximal segment 54. This provides a gradually tapering leading edge to balloon 52. This shape greatly eases entry of the balloon into narrow passages in stenoses.

Tip segment 60 is attached to spring guide 30 by means such as adhesive bonding. Interior 64 of balloon 52 is open to lumen 19 through torque cable 12, so that fluid may be used for balloon inflation/deflation in the standard method. In addition, the interstices between individual coil elements of torque cable 12 provide additional channels for inflation/deflation, as does the lumen between torque cable 12 and sheath 50.

A radiopaque marker band 70 is preferably mounted on wire 24 within the interior 64 of balloon 52. This marker band allows easier tracking of balloon 52 through standard flouroscopy methods.

Other embodiments of a torque cable constructed according to the present invention are illustrated in Figs. 5-8. As discussed above, it is preferable to taper the torque cable 12 so that maximum strength and torque transmission is achieved while having a lower profile and very flexible distal portion for entering coronary arteries.

In Fig. 5, the embodiment of torque cable 100 comprises first and second proximal coils 102 and 104 of wire of a first thickness. Inner and outer distal coils 106 and 108 are wound of finer wire than that of 102 and 104. Coils 106 and 108 are brazed to the distal end of coils 102 and 104 to taper cable 100 to a lower and more flexible profile. Coils 106 and 108 may also be a continued smaller diameter grind of coils 102 and 104, respectively.

In Fig. 6, another embodiment is illustrated in which cable 110 has a proximal section 112 consisting of three wire coils 114, 116 and 118. A smaller distal section 120 is either brazed to the distal end of section 112 or consists of two continued segments of any two of the three coils 114, 116 or 118. Distal section 120 consists of two wires 122 and 124. In this illustrated embodiment, all wires 114, 116, 118, 122, and 124 are of the same diameter. The transition to a more flexible and narrower profile is provided by the reduction in number of layers.

The embodiment of Fig. 7 consists of two layers of flat ribbon coil. This cable 130 has first coil 132 and second coil 134. Coils 132 and 134 are constructed in the manner of coil 18 of torque cable 12.

The embodiment of Fig. 8 illustrates a cable 140 having an outer ribbon coil 142 similar to ribbon coil 18. Inside of ribbon coil 142 are two coils wound of round wire, 144 and 146. A distal section 148 comprises a single coil 150 brazed to the distal end of coils 144 and 146 and a wire 152 brazed to the distal end of coil 150. Alternatively, single coil 150 could be a continuation of coil 144 by dropping off coil 146.

As is illustrated, various combinations of layers can be used to accomplish the desired flexibility and torque transmission for a particular application.

The torque cable 12 provides the "feel" desired by physicians who must insert an angioplasty catheter through various difficult curves in the arterial system. The torque cable provides a flexible catheter, which is sometimes characterized as limp. Unlike prior art devices that required a rigid wire for torque transmission, the torque cable 12 transmits torque from the proximal end to the distal end while still providing a flexible catheter which is easy to guide around curves.

While the prior art designs varied materials in an attempt to gain both torque transmission and flexibility, there were always sacrifices to be made in the design. In order to increase torque transmission, flexibility was lost. If a flexible catheter was desired, torque transmission was sacrificed. The preferred embodiments of the present invention achieve the desired feel by departing from the techniques of the prior art. Rather than increasing strength and rigidity in order to transmit torque, the torque cable bends and flexes in all directions. The torque cable is rigid only to the type of force which physicians wish transferred, that is torque applied to the catheter.

While the present invention has been disclosed in terms of fixed embodiments, it is clear that the invention has applicability to many catheter embodiments by those skilled in the art.

## Claims

1. A medical catheter for insertion in a bodily organ comprising:

a torque-transmitting cable having a proximal end and a distal end for insertion and having a longitudinal axis;

the cable comprising at least a first wire wound in a first coil at a first angle in a first direction, and a second wire wound in a second coil, generally concentric to the first coil in a second opposite direction at a second generally opposite angle;

the second coil being wound against the first coil, forming the torque-transmitting cable whereby rotational force applied at the proximal end results in generally equivalent rotational movement of the distal end; and

whereby the cable is flexible in all directions about the axis except for rotational torque means mounted generally adjacent the distal end for interacting with the bodily organ.

2. The catheter of claim 1 further comprising:

a third wire wound in a third coil around the second coil at a third angle generally opposite the second angle and generally in the first direction.

3. The catheter of claim 1 or 2, wherein the cable includes a central lumen.

4 The catheter of claim 3 further comprising stylet means slidable within the lumen for longitudinally stiffening the catheter.

5. The catheter of any preceding claim further comprising:

a generally fluid-tight non-torque-transmitting tube encompassing the cable.

6. The catheter of claim 5 wherein the means for interacting includes an angioplasty balloon mounted on the tube.

7. The catheter of claim 1, comprising a balloon made of inelastic material having a first frustroconical section tapering outward away from the sheath at a first angle to the longitudinal axis of the catheter;

a generally cylindrical midsection; and

a second frustroconical section tapering down to the distal end of the catheter at a second smaller angle to the longitudinal axis.

8. An angioplasty device comprising:

a torque-transmitting cable having a longitudinal axis with a proximal end and a distal end, a first wire coil wound at a first angle to the axis forming a central lumen, a second coil wound around the first coil at a second generally opposite angle to the axis, and a third coil wound about the second coil at a third angle generally opoposite the second angle, the three coils forming the cable so that rotational force applied to the proximal end is generally completely transmitted to the distal end, while the cable is flexible in all other directions;

a generally fluid-tight flexible, non-torque-transmitting sheath mounted over the cable;

a tip wire mounted on the distal end of the cable and extending distally from the cable;

a balloon mounted, at its proximal end, on the sheath having an interior open to the central lumen, the balloon having a distal end extending over the tip wire;

means for attaching the distal end of the balloon to the tip wire.

9. The device of claim 8, further comprising:

control means mounted on the proximal end of the cable for rotation of the cable and remainder of the catheter, so that the entire sheath and balloon rotate, avoiding twisting of the balloon.

FIG1.

FIG.4.

**FIG.2.**

40  41  43  44
3 →  39  38  4  42  4

**FIG.3.**

39  40  41

**FIG.5.**

104 102 100 106 108  50

**FIG.6.**

110 112 114 116 118  122  124 50

118  128  120

**FIG.7.**

130 132 134

**FIG.8.**

142 140  50  144 146 148 150  152

| Category | Citation of document with indication, where appropriate, of relevant passages | Relevant to claim | CLASSIFICATION OF THE APPLICATION (Int. Cl.⁵) |
|---|---|---|---|
| Y | EP - A1 - 0 254 701 (VERSAFLEX DELIVERY SYSTEMS INC.) <br> * Totality; especially column 3, lines 20-24; column 3, line 41 - column 4, line 9; column 6, lines 17-38,51-59; column 8, lines 33-36; claim 1; fig. 1,2,12 * | 1,3,5, 6 | A 61 M 25/01 |
| A | | 8,9 | |
| Y | EP - A2 - 0 274 412 (C.R. BARD, INC.) <br> * Column 3, lines 23-52; fig. 1,2 * | 1,3,5, 6 | |
| A | US - A - 4 719 924 (CRITTENDEN) <br> * Totality; especially column 2, line 60 - column 3, line 5 column 3, lines 33,34; column 4, lines 31-35; fig. 2,3 * | 1,8 | |
| D,A | US - A - 4 307 722 (EVANS) <br> * Totality; especially column 4, lines 1-37; fig. 1,2 * | 1,8 | TECHNICAL FIELDS SEARCHED (Int. Cl.⁵) <br><br> A 61 M 25/00 <br> A 61 M 29/00 |
| A | US - A - 4 737 153 (SHIMAMURA) <br> * Totality; especially column 2, lines 10-19,42-46; fig. 11 * | | |

The present search report has been drawn up for all claims

| Place of search | Date of completion of the search | Examiner |
|---|---|---|
| VIENNA | 03-07-1990 | VELINSKY-HUBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on or after the filing date
D : document cited in the application
L : document cited for other reasons

& : member of the same patent family, corresponding document

EPO Form 1503 03.82